# EUROPEAN PATENT SPECIFICATION

(11) **EP 1 499 284 B1**
(45) Date of publication and mention of the grant of the patent: **20.08.2008**
(21) Application number: 03725098.2
(22) Date of filing: 25.04.2003
(51) Int. Cl.: A61K 8/64, A61Q 13/00, A61K 38/08, A01K 67/02

(54) **BIOLOGICALLY ACTIVE COMPOUNDS FOR THE MODIFICATION OF BODILY ODOURS**
KÖRPERGERUCH VERÄNDERNDE BIOLOGISCH AKTIVE SUBSTANZEN
COMPOSES BIOACTIFS DESTINES A LA MODIFICATION DES ODEURS CORPORELLES

(30) Priority: 26.04.2002 EP 02009588
(43) Date of publication of application: 26.01.2005
(73) Proprietor: Max-Planck-Gesellschaft zur Förderung der Wissenschaften e.V., 80539 München (DE)
(72) Inventor: BOEHM, Thomas, 79104 Freiburg (DE); MILINSKI, Manfred, 24306 Plön (DE)
(74) Representative: Weiss, Wolfgang
(86) International application number: PCT/EP2003/004325
(87) International publication number: WO 2003/090705

(56) References cited:
- WO-A-98/07441
- WO-A1-95/28958
- DE-A- 10 021 579
- MILINSKI, M; WEDEKIND, C: "Evidence for MHC-correlated perfume preferences in humans" BEHAVIORAL ECOLOGY, vol. 12, no. 2, 2001, pages 140-149, XP001105765
- JACOB, S; MCCLINTOCK, MK; ZELANO, B; OBER, C: "Paternally inherited HLA alleles are associated with women's choice of male odor" NATURE GENETICS, vol. 30, no. 2, 22 January 2002 (2002-01-22), pages 175-179, XP001106682
- EGGERT, F; MÜLLER-RUCHHOLTZ, W; FERSTL, R: "Olfactory cues associated with the major histocompatibility complex" GENETICA, vol. 104, 1999, pages 191-197, XP001097711
- EGGERT, F ET AL: "The major histocompati- bility complex and the chemosensory signalling of individuality in humans" GENETICA, vol. 104, 1999, pages 265-273, XP001097710
- ZIEGLER, A: "Biology of Chromosome 6" DNA SEQUENCE - THE JOURNAL OF SEQUENCING AND MAPPING, vol. 8, no. 3, 1997, pages 189-201, XP001095837
- RAMMENSEE, HG ET AL: "SYFPEITHI: Database for MHC ligands and peptide motifs" IMMUNOGENETICS, SPRINGER VERLAG, BERLIN, vol. 50, no. 3-4, November 1999 (1999-11), pages 213-219, XP002183663 ISSN: 0093-7711
- MILINSKI M. ET AL.: 'Mate choice decisions of stickleback females predictably modified by MHC peptide ligands' PNAS vol. 102, no. 12, 22 March 2005, pages 4414 - 4418
- LEINDERS-ZUFALL T. ET AL.: 'MHC class I peptides as chemosensory signals in the vomeronasal organ' vol. 306, 05 November 2004, pages 1033 - 1037
- KIMOTO H. ET AL.: 'Sex-specific peptides from exocrine glands stimulate mouse vomeronasal sensory neurons' NATURE vol. 437, 06 October 2005, pages 898 - 901
- KIMOTO H. ET AL.: 'Sex- and strain-specific expression and vomeronasal activity of mouse ESP family peptides' CURRENT BIOLOGY vol. 17, 06 November 2007, pages 1879 - 1884
- CHAMERO P. ET AL.: 'Identification of protein pheromones that promote aggressive behaviour' NATURE vol. 450, 06 December 2007, pages 899 - 903

## Description

The invention relates to the use of MHC ligands, which are MHC-binding peptides, for the manufacture of an agent for modifying bodily odours. Particularly, the invention is applicable in the field of cosmetics and medicine.

Major histocompatibility complex (MHC) molecules present self and nonself peptides at the cell surface for recognition by T-lymphocytes. In natural populations, MHC genes are highly polymorphic(1); variability among MHC molecules leads to markedly different ligand specificities(2) and thereby controls resistance against pathogens and parasites. Several mechanisms have been proposed to explain the maintenance of high allelic diversity in MHC genes(3-5). MHC-based sexual selection strategies(6) are known to involve olfactory mechanisms in fish(7,8), mice(9), and possibly humans(10). Using male odours to guide their mate choice, females of the three-spined stickleback (Gasterosteus aculeatus) optimize the degree of MHC poly-morphism in their off-spring(7,8); however, the molecular nature of odour signals is unknown.

Body odour and reproductive behaviour are influenced by the highly polymorphic genes of the major histocompatibility complex (MHC) in many species(11,12). MHC molecules sample, for presentation at the cell surface, a certain subset of intracellular peptides; the chemical diversity of bound peptide populations is directly determined by the molecular structure of ligand binding grooves of MHC molecules(2). Therefore, information for individual recognition may be conferred either by the polymorphic MHC molecules themselves or by the chemical properties of their ligands. Whatever their molecular nature, in an experimental situation, odorant(s) that function in MHC-related sexual selection can be expected to have at least two properties. First, they should be able to modify the outcome of mate choice. Second, this effect should depend on the MHC status of mating partners. While MHC-related mating preferences for mice(9) and humans(10) are known for some time, this phenomenon was only recently described in fish(7,8). We have used this experimental system and the above criteria to identify potential odour signals.

We show that mate choice can be experimentally modified by synthetic peptides resembling MHC ligands. In most cases, gravid female sticklebacks preferred water of males that was supplemented with peptides to un-supplemented male water. However, when the addition of peptides to male water led to the rejection by the female, the combined MHC diversity (as estimated by class-IIB alleles) was significantly higher than in pairs preferring the peptide supplemented water. Thus, peptides function as odour signals for female sticklebacks when they assess the degree of MHC polymorphism of their potential mate.

The result that peptidic MHC ligands are capable of modifying bodily odours is completely surprising. So far peptides have not been taken into account as possible odorous substances due to their mass. Instead of this, it was assumed that the commensurate microorganism flora of an individual can be influenced by the MHC structure and thus via the microorganism MHC-specific low-molecular metabolic products are produced, and the latter represent part of the bodily odour(10,17,18).

DE 100 21 579 describes odour compositions derived from fragmented MHC molecules. It is not disclosed that peptidic MHC - ligands are capable to act as odorous substances.

Thus, the present invention describes the use of MHC ligands for modifying bodily odours.

The subject matter of the invention in a first embodiment is the non-therapeutic use of a composition for modifying bodily odours, said composition comprising at least one MHC-ligand as an active agent and optionally common cosmetic components. According to the invention, a MHC ligand is a peptide capable of binding to MHC-molecules and the concentration of the MHC - ligand is in the range of O.d nM to 100 µM.

A further embodiment of the invention is the use of an MHC ligand for the manufacture of a therapeutic composition for the reduction of the rate of abortions.

The invention is suitable for modifying bodily odours in animals, particularly in vertebrates such as fish, amphibia, reptiles, birds and mammals including human. The modification of bodily odours may result in modification of mate choices.

The MHC ligand may be an MHC class I or MHC class II ligand. Preferably, the MHC is an MHC class II ligand. The MHC ligand is a peptide, particularly a synthetic peptide. The peptide has preferably a length of from 5-20 amino acids, more preferably from 6-15 amino acids. Further, the peptide preferably exhibits anchor-amino acid residues required for the binding to allele-specific MHC molecules(2).

A preferred embodiment of the invention relates to the fields of cosmetics, particularly the manufacture of a perfume. Up to now the composing of perfumes was performed with natural or synthetic basic materials, based on empirical knowledge. In the light of the findings underlying the present invention the bodily odour can be influenced in a predetermined manner by means of adding defined peptidic MHC ligands or rather combinations of several MHC ligands to perfumes. Moreover, synthetic peptides can completely or at least partially substitute the costly substances present in perfumes so far, in order to achieve a cheaper and chemically defined composition of perfumes.

Cosmetic compositions according to the invention contain common cosmetic components, such as further active substances, carriers, diluents and/or adjuvants, apart from the MHC ligands. For example the cosmetic compositions may be formulated as liquids, gels, creams, slurries, ointments, aerosols, sprays, suspensions, powders etc. together with other ingredients, e.g. further odorants, auxiliaries, carriers and/or diluents. The compositions may be administered topically, orally or nasally or by any other suitable means. Preferably, the compositions are applied to skin and/or clothes of an individual in a manner that allows access of the compound to the environment, e.g. air space, enclosing the individual. The concentration of the active substance in the cosmetic compositions in the subnano to micromolar range from 0.01 nM to 100 µM.

In a further embodiment of the invention compositions for modifying bodily odours, which comprise MHC ligands as an active agent, may be used for therapeutic applications in human and veterinary medicine, e.g. for use in reproductive medicine. For example, it is known that couples having a high number of early abortions exhibit a great identity of their MHC types (reference 12 and citations therein). Thus, when employing different synthetic MHC peptides for one partner, a reduction of the rate of abortions may be achieved. Moreover, further applications in human medicine and veterinary medicine are conceivable, such as the increase of mating success in selected breeding.

The compositions for therapeutic applications contain the active substance in concentrations which are in the subnano- to micromolar range of 0.01 nM to 100 µM. Apart from this, the compositions can also contain pharmacologically acceptable carriers, diluents and/or adjuvants. The therapeutic compositions may be administered for instance by any means that allows access of the compound to the environment, e.g. air space, enclosing the individual, e.g. as described above for cosmetic compositions.

Furthermore, the invention shall be illustrated in detail by the following Figures and Examples.

### Figure 1

Peptides are recognized as odour cues in mate choice, **a**, Peptides with structural features of MHC ligands modify mate choice. Gravid female sticklebacks were tested with male water with (left panels) and without (right panels) addition of synthetic peptides. The excess time (in seconds) spent in each category is shown (0 indicates that the fish spent 300 seconds each in the two columns of the flow chamber). Five peptides (FAPGNYPAL, ASNENMETM, AAPDNRETF, SYFPEITHI and SYIPSAEKI) were added to a final concentration of 5nM. Overall, females (n = 24) spent a mean of 368.9 +/-36.5 sec. in the column containing the peptide mixture, **b**, MHC diversity of mating pairs determines the outcome of mate choice after peptide supplementation. The total number of different MHC class-IIB alleles in pairs of female and male fish was compared for groups of females preferring or rejecting peptide supplemented male water. MHC diversity is higher in pairs where the female rejected the peptide supplemented water. The box plots indicate median, quartiles and deciles.

### Table 1

Natural male odours and synthetic peptides display similar characteristics in mate choice experiments.

### EXAMPLE

### 1. Methods

### 1.1 MHC characterization

The characterization of MHC class-IIB genes was performed as described(7,29).

### 1.2 Sexual selection experiments

The source of sticklebacks has been described(7). The flow channel set-up to evaluate mate choice determines odour preference, as a precursor of mate choice. Water was taken from the tank of a single male per trial and used both as stimulus (after addition of peptide(s)) and control (after addition of solvent) water. Peptides were added to the bottle with stimulus water just before the experiment at a final concentration of 1mM; this becomes diluted to 5nM in the water column passing through the test compartment. The following nona-peptides were used, MHC specificities indicated in brackets: FAPGNYPAL (H2-K^{b}); ASNENMETM and AAPDNRETF (H2-D^{b}); SYFPEITHI and SYIPSAEKI (H2-K^{d}). Peptides were synthesized by solid-phase 9-fluorenylmethoxycarbonyl chemistry. In experiments using a single peptide, AAPDNRETF was used. All experiments were performed in double-blinded fashion as described. Statistical analysis was performed with JMP 4.

### 2. Results

The binding preferences for peptides of MHC molecules of sticklebacks are unknown; therefore synthetic peptides derived from endogenous mouse and viral proteins that are ligands for different mouse MHC molecules were used. Such peptides almost certainly appear foreign to sticklebacks irrespective of their MHC genotypes. Gravid females prefer water from a tank harbouring a male to that from a tank with fresh water (Experiment A, Table 1). Therefore, it was tested whether addition of peptide(s) to water from individual males would alter the outcome of mate choice. Gravid females were presented with water from single males with and without the addition of a mixture of five different peptides in the two columns of a flow-channel.

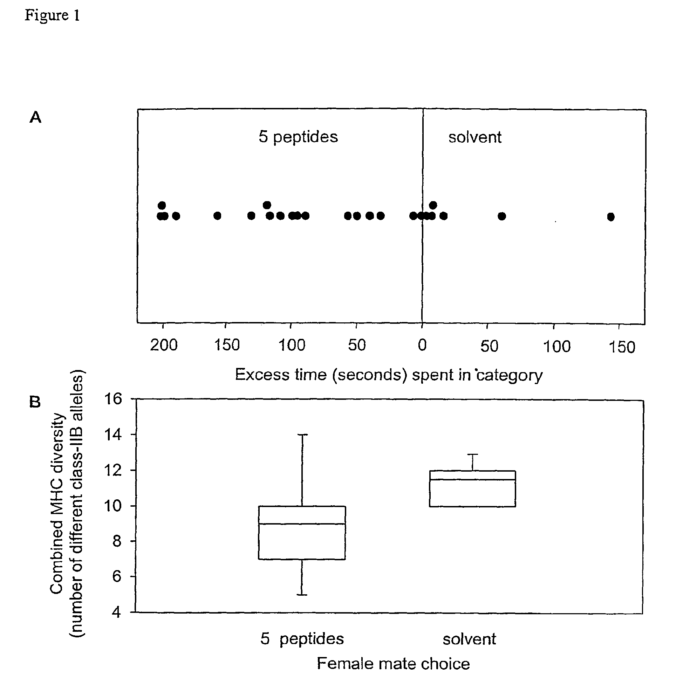

Most females preferred the spiked male water to its mock-supplemented version (Fig. 1 a), indicating that synthetic peptides are capable of modifying mate choice. Overall, the preference for peptide-supplemented male water was highly significant (368.9+/-36.5 sec. vs. 231.1 +/-36.5 sec.; matched-pair t-test: t = 3.8, n = 24, p = 0.001, two-tailed) (Table 1). However, in certain combinations of females and males, the female rejected the peptide-supplemented male water (Fig. 1a). Because female sticklebacks guide their mate choice towards an optimal degree of MHC polymorphism in their offspring(7,8), the experimental intervention would distort the preferred degree of MHC diversity during mate choice, if females accepted synthetic peptides as odour signals. The known genotypes at MHC class-IIB loci(7,13) were used in order to test this hypothesis. The results shown in Fig. 1b indicate that the combined MHC polymorphism represented in the pairs preferring plain male water is significantly higher than in the pairs preferring the water samples supplemented with peptides (11.5 (10, 12.25) different alleles vs. 9 (7, 10) different alleles; median (quartiles); Wilcoxon matched pair exact test: z =1.96, n = 21, p=0.046, two-tailed). It should be noted that mating pairs in the group rejecting the supplemented water would, on average, pass on to their offspring a number of detectably different class-IIB alleles closer to the average number observed in the population of wild-caught fish (5.8 + /-0.13; Ref. 7) than pairs from the group preferring the peptide supplement. The latter are thus attracted by the peptide supplemented water as this mimics increased MHC diversity in the male. This shows interaction of synthetic peptides and natural odour signals. It is also further evidence for the optimization strategy(7,8) used by sticklebacks during mate choice, the optimal number of different MHC class-IIB alleles being between 9 and 11.5.

When the above experiment was repeated with one peptide (Experiment C, Table 1), female sticklebacks again preferred the supplemented male water (327.7+/-24.8 sec. vs. 272.3+/-24.8 sec.; matched-pair t-test: t=2.2, n = 25, p= 0.035, two-tailed). Importantly, there is a strong trend that the excess time spent on the side of single peptide-supplemented male water is lower than that with five peptides (27.7+/-12.4 sec. vs. 68.9+/-18.2 sec.; matched-pair t-test: t=1.9, n = 49, p=0.066, two-tailed). Given a similar distribution of MHC diversity in mating pairs, this is to be expected, because a single peptide only marginally increases the presumptive odour complexity.

The properties of peptide signals perceived by female sticklebacks in odour-related mate choice were further investigated in the following experiments. First, the effect of peptides in plain tank water was determined. The results show that the addition of peptides to plain tank water is not attractive to female sticklebacks (Experiment B, Table 1). Rather, peptide(s) are only recognized in conjunction with male water (Experiments C and D, Table 1), indicating the presence of other, possibly invariant male odour signals. When non-gravid fish were exposed to male water with and without peptide, no preference was observed (Experiment E, Table 1), suggesting that gravidity is an important parameter to activate the recognition system. Importantly, experiments B and E (Table 1) also rule out the possibility that females recognize peptides out of the context of mate choice, i.e. as food signals etc.

Both the allele counting(7) and the dis-assortative mating(8) strategies used by sticklebacks require the assessment of the diversity of MHC peptide ligands. Although it is unknown at present whether peptides can be recognized by the olfactory system, a special class of olfactory receptors (termed V2R in mice) structurally are candidates for binding small peptides(14). Indeed, preliminary evidence indicates that receptors of the V2R class in mice interact with pheromonal components of undefined proteinaceous nature(15). For one V2R homologue in fish, high affinity binding to the amino acid arginine was found(16), but the effect of peptides or proteins was not investigated in this experiment. Due to their vast chemical diversity, peptides are much more likely to carry individual-specific information than the small compounds (for instance metabolites of amino acids) that have pre-viously been considered as signal molecules(17,18). Olfactory receptors are exquisitely sensitive, and recognize their ligands in the sub-nanomolar concentration range(14). Although thousands of chemically different peptides are presented by a particular MHC molecule on the cell surface(19), some peptides are much more frequent than others(20) and may be present in concentrations amenable to olfactory detection. For identification of MHC diversity, olfactory discrimination of such prevalent peptides may suffice.

MHC-bound peptides may become ligands for olfactory receptors in at least two ways. They may be released from MHC molecules and then bind to non-specific carrier proteins(21); in such a way, they may survive enzymatic degradation during transit in extracellular fluids and bodily secretions(22,23). In addition to the polymorphic MHC molecules as primary determinators of peptide diversity, polymorphic carrier proteins such as MUPs(24) may further modify the ligand repertoire(25,26) presented to olfactory receptors. An alternative but non-exclusive possibility is that peptides may remain bound to soluble MHC molecules or fragments thereof that have been detected in serum(27) and urine(28). Irrespective of the molecular nature of the carrier molecule(s), the recognition of carrier-bound peptides by olfactory receptors may occur in ways analogous to recognition by T-cell receptors.

In conclusion, the results suggest that peptides with structural features of MHC ligands constitute an individual-specific part of male odour in sticklebacks and also provide a conceptual frame-work to explain the results of previous experiments on MHC-related reproductive behaviour in other species, such as rodents and human.

**Table 1**

| Female | Experiment | Water source 1 | Water source 2 | Time spent on side of water source 1 (sec) | Time spent on side of water source 2 (sec) | n | p |
|---|---|---|---|---|---|---|---|
| Gravid | | | | | | | |
| | A | Fresh | Male | 229.2+/-46.8 | 365.2+/-46.8 | 14 | 0.01 |
| | B | Fresh + | Fresh+ 1 | 308.2+/-24.6 | 291.8+/-24.6 | 24 | 0.51 |
| | | solvent | peptide | | | | |
| | C | Male + solvent | Male + 1 peptide | 272.3+/-24.8 | 327.7+/-24.8 | 25 | 0.035 |
| | D | Male + solvent | Male + 5 peptides | 368.9+/-36.5 | 231.1+/-36.5 | 24 | 0.001 |

| Non-gravid | | | | | | | |
|---|---|---|---|---|---|---|---|
| | E | Male + | Male + 1 | 291.8+/-23.5 | 308.1+/-23.5 | 25 | 0.49 |
| | | solvent | peptide | | | | |

### REFERENCES

1. Little & Parham. Rev. Immunogenet. 1, 105-123 (1999).
2. Rammensee et al. MHC ligands and peptide motifs. (Landes Bioscience, Georgetown, 1997).
3. Apanius et al. Crit. Rev. Immunol. 17, 179-224 (1997).
4. Klein. Natural History of the Major Histocompatibility Complex (Wiley, New York, 1986).
5. Edwards & Hedrick. Trends Ecol. Evol. 13, 305-311 (1998).
6. Penn & Potts. Am. Nat. 153, 145-164 (1999).
7. Reusch et al. Nature 414, 300-302 (2001).
8. Aeschlimann et al., submitted for publication.
9. Yamazaki et al. J. Exp. Med. 144, 1324-1335 (1976).
10. Wedekind & Furi. Proc. R. Soc. London B. Biol. Sci. 264, 1471-1479 (1997).
11. Penn & Potts. Adv. Immunol. 69, 411-436 (1998).
12. Singh. Reproduction 121, 529-539 (2001).
13. Sato et al. Gastrosteus aculeatus. Mol. Mar. Biol. Biotechnol. 7, 221-231 (1998).
14. Firestein. Nature 413, 211-218 (2001).
15. Krieger et al. J. Biol. Chem. 274, 4655-4662 (1999).
16. Speca et al. Neuron 23, 487-498 (1999).
17. Singer et al. Proc. Natl. Acad. Sci. USA 94, 2210-2214 (1997).
18. Montag et al. Proc. Natl. Acad. Sci. USA 98, 9249-9254 (2001).
19. Bevan. Immunity 7, 175-178 (1997).
20. Yewdell & Bennink. Ann. Rev. Immunol. 17, 1-30 (1999).
21. Robertson et al. J. Chem. Ecol. 19, 1405-1416 (1993).
22. Hayakawa et al. J. Hered. 74, 453-456 (1983).
23. Utsumi et al. J. Neurobiol. 39, 227-236 (1999).
24. Robertson et al. Biochem. J. 316, 265-272 (1996).
25. Hurst et al. Nature 414, 631-634 (2001).
26. Brennan et al. Neuroscience 90, 1463-1470 (1999).
27. Wobst et al. Genetica 104, 275-283 (1998).
28. Singh et al. Nature 327, 161-164 (1987).
29. Binz et al. J. Fish Biol. 58, 887-890 (2001).

## Claims

1. Non-therapeutic use of a composition for modifying bodily odours said composition comprising at least one MHC ligand as an active agent and optionally common cosmetic components wherein the MHC ligand is a peptide capable of binding to MHC molecules and wherein the concentration of the MHC ligand is in the range of 0.01 nM to 100 µM.

2. The use of claim 1, wherein the non-therapeutic composition is a cosmetic composition.

3. The use of claim 2, wherein the cosmetic composition is a perfume.

4. The use of claim 1 for the increase of mating success in selected breeding.

5. Use of an MHC ligand for the manufacture of a therapeutic composition for the treatment of a medical indication selected from a reduction of the rate of abortions , wherein the MHC ligand is a peptide capable of binding to MHC molecules and wherein the concentration of the MHC ligand is in the range of 0.01 nM to 100 µM.

6. The use of claim 5 for use in human or veterinary medicine.

7. The use of any one of claims 1-6, wherein the MHC molecule is an MHC class I or an MHC class II molecule.

8. A cosmetic, non-therapeutic method for modifying bodily odours comprising applying a composition comprising at least one MHC ligand as an active agent and optionally common cosmetic components, to a subject in need of modifying bodily odours, wherein the MHC ligand is a peptide capable of binding to MHC molecules and wherein the concentration of the MHC ligand is in the range of 0.01 nM to 100 µM.

9. The method of claim 8 comprising application by topical means.

10. The method of claim 9 comprising application as an aerosol.

11. A cosmetic composition for modifying bodily odours comprising at least one MHC ligand as an active agent, and optionally common cosmetic components for topical administration in a manner that allows access of the active agent to the environment enclosing an individual, wherein the MHC ligand is a peptide capable of binding to MHC molecules and wherein the concentration of the MHC ligand is in the range of 0.01 nM to 100 µM.

12. A therapeutic composition for the treatment of a reproductive medical indication selected from a reduction of the rate of abortions comprising at least one MHC ligand as an active agent for topical administration in a manner that allows access of the active agent to the environment enclosing an individual wherein the MHC ligand is a peptide capable of binding to MHC molecules and wherein the concentration of the MHC ligand is in the range of 0.01 nM to 100 µM.

## Patentansprüche

1. Nicht-therapeutische Verwendung einer Zusammensetzung zur Modifikation von Körpergerüchen, wobei die Zusammensetzung mindestens einen MHC-Liganden als einen aktiven Wirkstoff umfasst und gegebenenfalls herkömmliche kosmetische Bestandteile, wobei der MHC-Ligand ein Peptid ist, das an MHC-Moleküle binden kann und wobei die Konzentration des MHC-Liganden im Bereich von 0,01 nM bis 100µM liegt.

2. Verwendung nach Anspruch 1, wobei die nicht-therapeutische Zusammensetzung eine kosmetische Zusammensetzung ist.

3. Verwendung nach Anspruch 2, wobei die kosmetische Zusammensetzung ein Parfum ist.

4. Verwendung nach Anspruch 1 zur Steigerung des Paarungserfolgs einer ausgewählten Züchtung.

5. Verwendung eines MHC-Liganden zur Herstellung einer therapeutischen Zusammensetzung zur Behandlung einer medizinischen Indikation ausgewählt aus einer Reduzierung der Fehlgeburtenrate, wobei der MHC-Ligand ein Peptid ist, das an MHC-Moleküle binden kann und wobei die Konzentration des MHC-Liganden im Bereich von 0,01 nM bis 100µM liegt.

6. Verwendung nach Anspruch 5 zur Verwendung in der Human- und Veterinärmedizin.

7. Verwendung nach einem der Ansprüche 1-6, wobei das MHC-Molekül ein MHC-Klasse I- oder ein MHC-Klasse II-Molekül ist.

8. Kosmetisches, nicht-therapeutisches Verfahren zur Modifikation von Körpergerüchen umfassend das Auftragen einer Zusammensetzung umfassend mindestens einen MHC-Liganden als einen aktiven Wirkstoff und gegebenenfalls herkömmliche kosmetische Bestandteile, auf ein Individuum, das eine Modifikation des Körpergeruchs benötigt, wobei der MHC-Ligand ein Peptid ist, das an MHC-Moleküle binden kann und wobei die Konzentration des MHC-Liganden im Bereich von 0,01 nM bis 100µM liegt.

9. Verfahren nach Anspruch 8 umfassend eine topische Anwendung.

10. Verfahren nach Anspruch 9 umfassend eine Anwendung als ein Aerosol.

11. Kosmetische Zusammensetzung zur Modifikation von Körpergerüchen umfassend mindestens einen MHC-Liganden als einen aktiven Wirkstoff und gegebenenfalls herkömmliche kosmetische Bestandteile zur topischen Verabreichung, die es ermöglicht, dass der aktive Wirkstoff Zugang zur Umgebung, einschließlich Individuen, erhält, wobei der MHC-Ligand ein Peptid ist, das an MHC-Moleküle binden kann und wobei die Konzentration des MHC-Liganden im Bereich von 0,01 nM bis 100µM liegt.

12. Therapeutische Zusammensetzung zur Behandlung einer reproduktiven medizinischen Indikation betreffend die Fortpflanzung ausgewählt von einer Reduzierung der Fehlgeburtenrate umfassend mindestens einen MHC-Liganden als einen aktiven Wirkstoff zur topischen Verabreichung, die es ermöglicht, dass der aktive Wirkstoff Zugang zur Umgebung, einschließlich Individuen, erhält, wobei der MHC-Ligand ein Peptid ist, das an MHC-Moleküle binden kann und wobei die Konzentration des MHC-Liganden im Bereich von 0,01 nM bis 100µM liegt.

## Revendications

1. Utilisation non thérapeutique d'une composition destinée à modifier des odeurs corporelles, ladite composition comprenant au moins un ligand du CMH en tant qu'agent actif et, facultativement, des composants cosmétiques classiques, dans laquelle le ligand du CMH est un peptide capable de se lier à des molécules du CMH et dans laquelle la concentration du ligand du CMH est dans la plage de 0,01 nM à 100 µM.

2. Utilisation selon la revendication 1, dans laquelle la composition non thérapeutique est une composition cosmétique.

3. Utilisation selon la revendication 2, dans laquelle la composition cosmétique est un parfum.

4. Utilisation selon la revendication 1, pour accroître la réussite des accouplements en reproduction sélectionnée.

5. Utilisation d'un ligand du CMH pour la fabrication d'une composition thérapeutique destinée au traitement d'une indication médicale choisie parmi une réduction du taux d'avortement, dans laquelle le ligand du CMH est un peptide capable de se lier à des molécules du CMH et dans laquelle la concentration du ligand du CMH est dans la plage de 0,01 nM à 100 µM.

6. Utilisation selon la revendication 5, pour un usage en médecine humaine ou vétérinaire.

7. Utilisation selon l'une quelconque des revendications 1 à 6, dans laquelle la molécule du CMH est une molécule du CMH de classe I ou de classe II.

8. Procédé cosmétique non thérapeutique permettant de modifier des odeurs corporelles, comprenant l'application d'une composition comprenant au moins un ligand du CMH en tant qu'agent actif et, facultativement, des composants cosmétiques classiques sur un sujet dont il faut modifier les odeurs corporelles, le ligand du CMH étant un peptide capable de se lier à des molécules du CMH et la concentration du ligand du CMH étant dans la plage de 0,01 nM à 100 µM.

9. Procédé selon la revendication 8, comprenant l'application par des moyens topiques.

10. Procédé selon la revendication 9, comprenant l'application sous la forme d'un aérosol.

11. Composition cosmétique destinée à modifier des odeurs corporelles, comprenant au moins un ligand du CMH en tant qu'agent actif et, facultativement, des composants cosmétiques classiques pour une administration topique d'une manière qui permet l'accès de l'agent actif à l'environnement entourant un individu, le ligand du CMH étant un peptide capable de se lier à des molécules du CMH et la concentration du ligand du CMH étant dans la plage de 0,01 nM à 100 µM.

12. Composition thérapeutique destinée au traitement d'une indication médicale reproductive choisie parmi une réduction du taux d'avortement, comprenant au moins un ligand du CMH en tant qu'agent actif, pour une administration topique d'une manière qui permet l'accès de l'agent actif à l'environnement entourant un individu, dans laquelle le ligand du CMH est un peptide capable de se lier à des molécules CMH et dans laquelle la concentration du ligand du CMH est dans la plage de 0,01 nM à 100 µM.
